# EUROPEAN PATENT APPLICATION

(11) **EP 4 830 535 A1**
(43) Date of publication of application: **28.10.2026**
(21) Application number: 24907553.2
(22) Date of filing: 20.12.2024
(51) Int. Cl.: A23L 7/10, A01H 6/46, A21D 2/36, A21D 13/00, C07K 14/415, C12N 15/29

(54) **WHEAT FLOUR PREPARED FROM NOVEL WHEAT**

(30) Priority: 22.12.2023 JP 2023216534
(71) Applicant: Nippn Corporation, Tokyo 102-0083 (JP); National Agriculture and Food Research Organization, Tsukuba-shi, Ibaraki 305-8517 (JP)
(72) Inventor: SHIMBATA Tomoya, Atsugi-shi, Kanagawa 243-0041 (JP); INOKUMA Takayuki, Atsugi-shi, Kanagawa 243-0041 (JP); TANAKA Tomoki, Atsugi-shi, Kanagawa 243-0041 (JP); TOUGOU Makoto, Tsukuba-shi, Ibaraki 305-8518 (JP); HATTA Koichi, Sapporo-shi, Hokkaido 062-8555 (JP); WATANABE Mikiko, Koshi-shi, Kumamoto 861-1192 (JP); TAIRA Masato, Koshi-shi, Kumamoto 861-1192 (JP); NAKATA Masaru, Koshi-shi, Kumamoto 861-1192 (JP); MATSUNAKA Hitoshi, Sapporo-shi, Hokkaido 062-8555 (JP); TERASAWA Yohei, Sapporo-shi, Hokkaido 062-8555 (JP)
(74) Representative: Eisenführ Speiser
(86) International application number: PCT/JP2024/045256
(87) International publication number: WO 2025/135170

(57) **Abstract**

The present invention has an object to provide a food product having improved texture in terms of stickiness and meltability in the mouth compared to food products made using GA-SX wheat flour.

The above object is achieved by introducing a d-type allele of a high molecular weight glutenin gene *Glu-D1* into GA-SX wheat, which does not lack GBSSI-A1 enzyme activity, lacks GBSSI-B1 and GBSSI-D1 enzyme activity, and lacks enzyme activity of any two of SSIIa-A1, SSIIa-B1, and SSIIa-D1.

## Description

### Technical Field

The present invention relates to wheat flour prepared from novel wheat usable as a raw material for wheat flour compositions for use in food production, and more specifically from wheat which lacks enzyme activity of two granule bound starch synthases I (GBSSI) and two starch synthases IIa (SSIIa), and in which a high molecular weight glutenin gene *Glu-D1* is a d-type allele.

### Background Art

Wheat flour is widely used as an ingredient in various processed foods that are consumed daily as meals and snacks. Many of these processed foods are produced through a heating process, but start changing in quality immediately after the heating process is completed. Taking white bread as an example, the texture immediately after baking is a very soft and strongly moist texture. However, after several hours or days, the texture changes to a hard and dry texture. This phenomenon is generally calledstaling, which is referred to as deterioration in the present invention. Since the progression of deterioration directly affects deliciousness, to retard this progression is an important problem for the food industry.

To solve this problem, wheat flour (GA-SX wheat flour) was developed which is obtained by milling wheat which does not lack a granule bound starch synthase I (GBSSI)-A1 responsible for amylose synthesis among enzymes involved in the biosynthesis of wheat seed endosperm starch, lacks GBSSI-B1 and D enzyme activity, and lacks enzyme activity of any two of starch synthases IIa (SSIIa)-A1, B1, and D1 involved in the elongation of side chains of amylopectin (Patent Literature 1: Japanese Patent No. 6226165). As a result of having a structure in which the amylose content is low and the side chains of amylopectin are shortened, the starch of this wheat is characterized by a slow rate of starch retrogradation. Consequently, this wheat is enabled to provide a soft texture, retard the deterioration progression after production, and maintain the deliciousness for a long time. Such effects are particularly excellent in GA-SA wheat flour (wheat flour obtained by milling wheat which does not lack GBSSI-A1, lacks GBSSI-B1 and D1 enzyme activity, does not lack SSIIa-A1, and lacks SSIIa-B1 and D1).

However, foods made using the GA-SX wheat flour have drawbacks of becoming sticky during chewing and having poor meltability in the mouth, and these drawbacks are particularly noticeable in bakery foods such as white bread. This stickiness and poor meltability in the mouth are presumably because the starch contained in the GA-SX wheat has the aforementioned structure. In wheat, a type in which all three GBSSI are functional contains about 25% amylose, but a mutant lacking one of them contains 23 to 24% amylose (decreased by 1 to 2%), a mutant lacking two of them contains about 20%, and a mutant lacking all the three is waxy starch containing no amylose. This difference in amylose content affects the gelatinization and retrogradation properties of the starch. Regarding gelatinized starch, it is known that amylose retrogrades faster than amylopectin in a short term. Therefore, starch with a high amylose content can harden easily and fast due to retrogradation, while waxy starch containing no amylose can maintain its soft state. For example, in the case of bread, the properties of the starch affect the texture, resulting in the texture strongly characterized in that it becomes softer as the amylose content becomes lower, but on the other hand, becomes poorer in the stickiness and meltability in the mouth as the proportion of amylopectin increases. The GA-SX wheat has an amylose content of 20% or less (Non Patent Literatures 1 and 2), which is thought to be the reason why the GA-SX wheat results in the texture poor in the stickiness and meltability in the mouth.

Meanwhile, glutenin and gliadin are major proteins in wheat and form viscoelastic gluten when they come into contact with water in their coexistence. Glutenin polymerizes into a giant polymer by forming disulfide bonds between its molecules, and is involved in the elasticity of wheat flour dough (dough strength). Gliadin exists in a state of monomers weakly bonded by hydrogen bonds or the like, and is involved in the extensibility of wheat flour dough. Glutenin is broadly classified into high molecular weight glutenin and low molecular weight glutenin. The high molecular weight glutenin is encoded at *Glu-A1*, *B1*, and *D1* gene loci located on the long arms of wheat chromosomes 1A, 1B, and 1D, while the low molecular weight glutenin is encoded at *Glu-A3*, *B3*, and *D3* gene loci located on the short arms of the same chromosomes 1A, 1B, and 1D.

Numerous alleles are known for each of these six gene loci. It is known that the molecular weight and expression level of an encoded subunit differ depending on an allele type, which affects the secondary processing properties of wheat flour. G*lu-D1* alleles include types such as a (having a high molecular weight glutenin subunit pair "2+12"), c (4+12), d (5+10), and f (2.2+12) (Non Patent Literature 3). Among these, the *Glu-D1d* allele is known to produce a greater effect of strengthening a dough during bread making and expanding the bread volume than the other *Glu-D1* alleles (Non Patent Literatures 4 and 5). Similarly, regarding *Glu-B3*, it is known that allele types such as *Glu-B3b*, *B3h*, and *B3i* produce a greater effect of strengthening a dough during bread making than the other *Glu-B3* allele types (such as c, j, and ae types) (Non Patent Literatures 4 and 6).

As a result of diligently studying how to obtain wheat flour with even higher quality, the present inventors unexpectedly found that the drawbacks of GA-SX wheat flour can be overcome by introducing a *Glu-D1d* allele into the GA-SX wheat, thereby leading to the completion of the present invention. In addition, the present inventors found that the drawbacks of the GA-SX wheat flour could be further eliminated by introducing a *Glu-B3b* or *Glu-B3h* allele, thereby leading to the completion of the present invention.

### Citation List

### Patent Literature

Patent Literature 1: Japanese Patent No. 6226165

### Non Patent Literatures

Non Patent Literature 1: Inokuma et al., J. Agric. Food Chem. 2016, 64, 4, 941-947
Non Patent Literature 2: Inokuma et al., J. Agric. Food Chem. 2021, 69, 7, 2271-2278
Non Patent Literature 3: Payne and Lawrence, Cereal Research Communications, (1983) 11, 1, 29-35
Non Patent Literature 4: Tatsuya Ikeda, Journal of the Japan Society for Food Chemistry and Technology (2017), 64, 3, 171-176
Non Patent Literature 5: Takata et al., Breeding Science (2000) 50, 303-308
Non Patent Literature 6: Zhang et al., 2012, BMC Plant Biology, 12: 243
Non Patent Literature 7: Vrinten et al., Mol. Gen. Genet. (1999), 261: 463-471
Non Patent Literature 8: Saito et al., Mol. Breeding, 2009, 23, 209-217
Non Patent Literature 9: Shimbata et al., 2005, Theor. Appl. Genet., 111, 6, 1072-1079
Non Patent Literature 10: Osakabe et al., Proc. Natl. Acad. Sci. USA (2010) 107(26): 12034-12039
Non Patent Literature 11: Ishikawa et al., (2005) Research Report of Tohoku Agricultural Research Center, 27-37
Non Patent Literature 12: R.B. Gupta and K. W. Shepherd, Theor Appl. Genet. (1990) 80: 65-74
Non Patent Literature 13: Kojima et al., (2017) Report of the National Agriculture and Food Research Organization, Crop Development Center 1, 1-13
Non Patent Literature 14: Wang et al., Theor. Appl. Genet. 2009, 118: 525-539
Non Patent Literature 15: Fukuda et al., (2010) Breeding Research 12, 87-95
Non Patent Literature 16: Masanori Inagaki (2001) Journal of Agricultural, Forestry and Fisheries Technology Research, Vol. 24, No. 12, 44-49
Non Patent Literature 17: Nakamura et al., Genome, 2002, 45, 1150-1156

### Summary of Invention

### Problems to be solved by the invention

The present invention has an object to provide a food product improved in stickiness and meltability in the mouth compared to food products made from GA-SX wheat flour.

### Means for solution of the problems

As a result of diligent studies to achieve the above object, the present inventors found that the drawbacks of GA-SX wheat flour can be overcome by introducing a d-type allele of a high molecular weight glutenin gene *Glu-D1* into GA-SX wheat (wheat that does not lack GBSSI-A1 enzyme activity, lacks GBSSI-B1 and GBSSI-D1 enzyme activity, and lacks enzyme activity of any two of SSIIa-A1, SSIIa-B1, and SSIIa-D1), thereby leading to the completion of the present invention. The present inventors also found that the drawbacks of GA-SX wheat flour can be further overcome by further introducing a b-type allele or an h-type allele of a low molecular weight glutenin gene *Glu-B3*, thereby leading to the completion of the present invention.

In sum, the present invention encompasses the following aspects.
[1] Wheat flour obtained by milling harvested wheat which does not lack GBSSI-A1 enzyme activity, lacks GBSSI-B1 and GBSSI-D1 enzyme activity, lacks enzyme activity of any two of SSIIa-A1, SSIIa-B1 and SSIIa-D1, and in which a high molecular weight glutenin gene *Glu-D1* is a d-type allele (GA-SX/GD1d wheat flour).
[2] The wheat flour according to [1], in which a low molecular weight glutenin gene *Glu-B3* is a b-type allele (GA-SX/GD1d/GB3b wheat flour).
[3] The wheat flour according to [1], in which a low molecular weight glutenin gene *Glu-B3* is an h-type allele (GA-SX/GD1d/GB3h wheat flour).
[4] A cereal flour composition comprising the wheat flour according to any one of [1] to [3].
[5] A method for producing a food product using the wheat flour according to any one of [1] to [3].
[6] The method according to [5], in which the food product is a bakery food product.

### Advantageous Effects of Invention

A food product made using GA-SX wheat flour exhibits reduced hardening (deterioration) during a storage period of several days after production. However, this product may provide undesirable texture such as poor meltability in the mouth and stickiness. By using wheat flour prepared from the wheat of the present invention, it is possible to obtain a product with improved texture while maintaining a level of deterioration reduction comparable to conventional GA-SX wheat flour.

### Description of Embodiments

Wheat used in the present invention is wheat (GA-SX/GD1d wheat) which does not lack GBSSI-A1 enzyme activity, lacks GBSSI-B1 and GBSSI-D1 enzyme activity, lacks enzyme activity of any two of SSIIa-A1, SSIIa-B1 and SSIIa-D1, and in which a high molecular weight glutenin gene *Glu-D1* is a d-type allele. The preferable wheat is wheat in which additionally a low molecular weight glutenin gene *Glu-B3* is a b-type allele (GA-SX/GD1d/GB3b wheat) or wheat in which additionally the low molecular weight glutenin gene *Glu-B3* is an h-type allele (GA-SX/GD1d/GB3h wheat).

Common wheat is an allohexaploid and its chromosomes are composed of three genomes, namely, A, B, and D genomes, each consisting of homoeologous chromosomes 1 to 7 (1A to 7A, 1B to 7B, and 1D to 7D).

"GBSSI" is a granule bound starch synthase involved in the synthesis of amylose contained in wheat endosperm starch, and is also called Waxy (Wx). GBSSI(Wx)-A1, GBSSI(Wx)-B1, and GBSSI(Wx)-D1 are encoded by genes located on chromosomes 7A, 4A, and 7D, respectively. For each of them, mutants lacking the enzyme function are known, and the amylose content differs depending on a combination of mutants.

"SSIIa" is an enzyme involved in the elongation of side chains (branched chains) of amylopectin in wheat endosperm starch. Similar to GBSSI, SSIIa-A1, B1, and D1 are functional in common wheat and defective mutants of each of them are also known. SSIIa-A1, SSIIa-B1, and SSIIa-D1 are encoded by genes located on chromosomes 7A, 7B, and 7D, respectively. If one of the three enzymes is deficient, the side chains of amylopectin are slightly shortened. If two of the enzymes are deficient, the degree of the chain shortening increases. If all the three enzymes are deficient, the side chains are shortened the most, resulting in high-amylose wheat with an amylose content exceeding 30% as a secondary effect.

The phrase "lack enzyme activity" means that a protein with normal enzyme activity is not functioning in a wheat plant, and preferably means that a protein with normal enzyme activity is not expressed. Specific lacking forms include mutations in gene sequences (referring to mutations such as a substitution, a deletion, an insertion, an inversion, and a translocation of one or more bases, including a deletion of an entire gene region), a defect in mRNA transcription, a defect in protein translation, an inhibition of enzyme activity within a wheat plant, and so on. Any lacking form may be taken as long as the enzyme activity is deleted or reduced to less than 10%, preferably less than 5%, and more preferably less than 1% of the wild-type enzyme activity.

In the present specification, "wheat which does not lack GBSSI-A1 enzyme activity, lacks GBSSI-B1 and GBSSI-D1 enzyme activity, and lacks enzyme activity of any two of SSIIa-A1, SSIIa-B1, and SSIIa-D1" is referred to as "GA-SX wheat".

As the GA-SX wheat, there are the following types of wheat depending on a combination of two types of SSIIa lacking enzyme activity:
GA-SA wheat: wheat that does not lack GBSSI-A1 enzyme activity, lacks GBSSI-B1 and GBSSI-D1 enzyme activity, does not lack SSIIa-A1 enzyme activity, and lacks SSIIa-B1 and SSIIa-D1 enzyme activity;
GA-SB wheat: wheat that does not lack GBSSI-A1 enzyme activity, lacks GBSSI-B1 and GBSSI-D1 enzyme activity, does not lack SSIIa-B1 enzyme activity, and lacks SSIIa-A1 and SSIIa-D1 enzyme activity; and
GA-SC wheat: wheat that does not lack GBSSI-A1 enzyme activity, lacks GBSSI-B1 and GBSSI-D1 enzyme activity, does not lack SSIIa-D1 enzyme activity, and lacks SSIIa-A1 and SSIIa-B1 enzyme activity.

The GA-SX wheat is wheat that can be produced by a known method, for example, the method described in Japanese Patent Application Publication No. 2013-188206.

As examples of so-called wild-type genes that do not lack enzyme activity, sequences of *GBSSI-A1*, *B1*, *D1*, and *SSIIa-A1*, *B1*, *D1* (genomic DNAs and proteins) are publicly known and registered in GenBank with the following accession numbers, respectively. These sequences are shown in the sequence listing as specified in Table 1 below.

**[Table 1]**

| | GenBank Accession No. | Sequence Listing |
|---|---|---|
| *GBSSI-A1 (Wx-A1)* | AB019622 | SEQ ID NO: 1 (Genomic DNA) |
| | | SEQ ID NO: 2 (Protein) |
| *GBSSI-B1 (Wx-B1)* | AB019623 | SEQ ID NO: 3 (Genomic DNA) |
| | | SEQ ID NO: 4 (Protein) |
| *GBSSI-D1 (Wx-D1)* | AB019624 | SEQ ID NO: 5 (Genomic DNA) |
| | | SEQ ID NO: 6 (Protein) |
| *SSIIa-A1* | AB201445 | SEQ ID NO: 7 (Genomic DNA) |
| | | SEQ ID NO: 8 (Protein) |
| *SSIIa-B1* | AB201446 | SEQ ID NO: 9 (Genomic DNA) |
| | | SEQ ID NO: 10 (Protein) |
| *SSIIa-D1* | AB201447 | SEQ ID NO: 11 (Genomic DNA) |
| | | SEQ ID NO: 12 (Protein) |

These sequences are examples of wild-type sequences. Some varieties of naturally occurring wheat (including improved wheat varieties) have comparable activity of enzyme proteins but are slightly different in the base sequence or amino acid sequence. In the present invention, the terms "*GBSSI-A1* gene" and "GBSSI-A1 protein" include not only those with sequences completely identical to the base or amino acid sequences specified in the sequence listing, but also those with sequences containing natural mutations that do not impair their enzyme activity. The same applies to the other enzymes. Such a natural mutant sequence typically has 90% or more, for example, 95% or more or 98% or more identity with each base or amino acid sequence specified in the sequence listing. In the present specification, these *GBSSI-A1*, *B1*, *D1* genotypes and *SSIIa-A1*, *B1*, *D1* genotypes are referred to as *GBSSI-A1a*, *B1a*, *D1a* alleles and *SSIIa-A1a*, *B1a*, *D1a* alleles.

In contrast to these wild types, known examples each lacking enzyme activity are described below. As for GBSSI-A1, a mutant is known in which the expression of the coding protein GBSSI-A1 is deleted in the wild-type *GBSSI-A1* (*Wx-A1*) gene sequence due to a gene mutation with a deletion of 23 base pairs and an insertion of a different sequence of 4 bases at the junction site between the first exon and the following intron (Non Patent Literature 7). In the present specification, this mutant is referred to as *GBSSI-A1b* allele.

As for GBSSI-B1, a mutant is known in which the wild-type *GBSSI-B1* (*Wx-B1*) has a deletion of the entire gene region from the start codon to the stop codon (Non Patent Literature 8). In the present specification, this mutant is referred to as *GBSSI-B1b* allele.

As for GBSSI-D1, a mutant is known in which the wild-type *GBSSI-D1* (*Wx-D1*) gene sequence loses the expression of GBSSI-D1 due to a gene mutation with a deletion of 588 bases around the stop codon and an insertion of a different sequence of 12 bases (Non Patent Literature 7). In the present specification, this mutant is referred to as *GBSSI-D1b* allele.

As for SSIIa-A1, a mutant is known in which the wild-type *SSIIa-A1* gene sequence loses the expression of SSIIa-A1 due to a gene mutation with a deletion of a 289-base region containing the start codon and an insertion of a different sequence of 8 bases (Non Patent Literature 9). In the present specification, this mutant is referred to as *SSIIa-A1b* allele.

As for SSIIa-B1, a mutant is known in which the wild-type *SSIIa-B1* gene sequence has an insertion of 175 bases into exon 8 and this insertion results in generation of a stop codon, leading to a loss of the expression of normal SSIIa-B enzyme protein (Non Patent Literature 9). In the present specification, this mutant is referred to as *SSIIa-B1b* allele.

As for SSIIa-D1, a mutant is known in which the wild-type *SSIIa-D1* gene sequence loses the expression of normal SSIIa-D enzyme protein due to a gene mutation with a deletion of 63 bases around the junction region between exon 5 and the subsequent intron (Non Patent Literature 9). In the present invention, this mutant is referred to as *SSIIa-D1b* allele.

"Glutenin" is a main protein in wheat and forms viscoelastic gluten when it comes into contact with water in the coexistence with gliadin. Glutenin polymerizes into a giant polymer by forming disulfide bonds between its molecules, and is involved in the elasticity of wheat flour dough (dough strength). Gliadin exists in a state of monomers weakly bonded by hydrogen bonds or the like, and is involved in the extensibility of wheat flour dough. Glutenin is broadly classified into high molecular weight glutenin and low molecular weight glutenin.

The "high molecular weight glutenin" is encoded at the *Glu-A1*, *B1*, and *D1* gene loci located on the long arms of the wheat chromosomes 1A, 1B, and 1D, while the "low molecular weight glutenin" is encoded at the *Glu-A3*, *B3*, and *D3* gene loci located on the short arms of the same chromosomes 1A, 1B, and 1D. Numerous alleles are known for each of these six gene loci. It is known that the molecular weight and expression level of an encoded subunit differ depending on an allele type, which affects the secondary processing properties of wheat flour.

The wheat used in the present invention is GA-SX wheat and the high molecular weight glutenin gene *Glu-D1* is the d-type allele (GA-SX/GD1d). The wheat is preferably such that, additionally, the low molecular weight glutenin gene *Glu-B3* is a b-type allele (GA-SX/GD1d/GB3b wheat) or the low molecular weight glutenin gene *Glu-B3* is an h-type allele (GA-SX/GD1d/GB3h wheat). In another embodiment, it is GA-SX wheat in which the high molecular weight glutenin gene *Glu-D1* is the d-type allele, and preferably additionally, the low molecular weight glutenin gene *Glu-B3* is an i-type allele (GA-SX/GD1d/GB3i wheat).

Since the wheat used in the present invention is GA-SX wheat, the genetic traits related to the lack of GBSSI and SSIIa enzyme activity may be produced by crossing known wheat varieties that lack any combination of the six enzymes. Radiation treatment (γ rays, β rays, X rays, neutrons, or the like), chemical treatment (ethyl methanesulfonic acid or the like), or any other mutagenic treatment may be performed and desired enzyme-deficient plants may be selected and used for crossbreeding. In addition, various methods for producing transformants of monocotyledonous plants are known, and also genetic engineering techniques for deleting the function of a target gene are known. For example, there is a method for inhibiting the expression of a target gene by RNAi or the antisense method, and also a gene disruption method is known which destroys only a target gene in plants (Non Patent Literature 10). Therefore, the wheat may also be created by such a genetic engineering technique. In combination with the lack of the enzyme activity of two GBSSI and two SSIIa as described above, a d-type allele of the high molecular weight glutenin gene *Glu-D1* is introduced. Preferably, a b-type or an h-type allele of the low molecular weight glutenin gene *Glu-B3* is additionally introduced. The wheat may be created by first creating the wheat lacking the enzyme activity of two GBSSI and two SSIIa, and then introducing desired allele types into the high molecular weight glutenin gene *Glu-D1* and preferably additionally into the glutenin gene *Glu-B3*. Instead, the wheat may be created by selecting wheat varieties such that the high molecular weight glutenin gene *Glu-D1* and preferably also the glutenin gene *Glu-B3* have the desired allele types in the process of creating the wheat lacking the enzyme activity of two GBSSIs and two SSIIa.

The *Glu-D1d* allele is based on the classification and nomenclature proposed in Non Patent Literature 3. Whether or not wheat possesses the *Glu-D1d* allele can be identified by detecting a specific base sequence within the *Dx5* gene (GenBank accession No. X12928: SEQ ID NO: 13), which encodes subunit 5 in the *Glu-D1d* allele. For this identification method, it is possible to use the PCR method using Dx_F, Dx 5_F, and Dx_R primers (Dx_F: SEQ ID NO: 20, Dx 5_F: SEQ ID NO: 21, Dx_R: SEQ ID NO: 22) described in Non Patent Literature 11. Alternatively, the identification may be made by: amplifying a region containing this specific mutation using appropriately designed primers; and analyzing and confirming that the resultant gene sequence matches this specific sequence. Instead, a band specific to the *Glu-D1d* allele can be identified by extracting a fraction containing high molecular weight glutenin protein from wheat, separating the protein by SDS-PAGE, and comparing the protein with that of known wheat (Non Patent Literature 3).

The *Glu-B3b*, *B3h*, and *B3i* alleles are based on the classification and nomenclature proposed in Non Patent Literature 12. The following methods can be used to identify these alleles.

Whether or not wheat possesses the *Glu-B3b* allele can be determined by examining whether or not the wheat possesses a sequence specific to *Glu-B3b* in the gene sequence specified in Non Patent Literature 14 (GenBank Accession No. EU369719: SEQ ID NO: 14). This identification may be made by: performing PCR using SB2F and SB2R primers described in Non Patent Literature 14 (SB2F: SEQ ID NO: 23 and SB2R: SEQ ID NO: 24); and examining whether or not an amplified fragment with a desired length is obtained. Alternatively, the identification may be made by: performing PCR using LB1F and LB1R primers or LB4F and LB4R primers described in Non Patent Literature 14 (LB1F: SEQ ID NO: 25, LB1R: SEQ ID NO: 26, LB4F: SEQ ID NO: 27, and LB4R: SEQ ID NO: 28); and analyzing the gene sequence of the resulting amplified fragment to examine whether or not the gene sequence matches the *Glu-B3b* gene sequence (GenBank Accession No. EU369700: SEQ ID NO: 15 or EU369719: SEQ ID NO: 14). Alternatively, a band specific to the *Glu-D1d* allele can be identified by: extracting a fraction containing low molecular weight glutenin protein from wheat, separating the protein by SDS-PAGE; and comparing the protein with that of known wheat (Non Patent Literature 12). Known wheat varieties containing the *Glu-B3b* allele include "Takune Komugi", "Nanbu Komugi", and so forth (Non Patent Literature 13).

Whether or not wheat possesses the *Glu-B3h* allele can be identified by: examining whether the wheat possesses a sequence specific to *Glu-B3h* in the gene sequence described in Non Patent Literature 14 (GenBank Accession No. EU369717: SEQ ID NO: 16). This identification may be made by: performing PCR using SB8F and SB8R primers (SB8F: SEQ ID NO: 29 and SB8R: SEQ ID NO: 30) described in Non Patent Literature 14 and checking whether or not an amplified fragment with a desired length is obtained. Alternatively, the identification may be made by: performing PCR using LB3F and LB3R primers (LB3F: SEQ ID NO: 31 and LB3R: SEQ ID NO: 32) described in Non Patent Literature 14; analyzing the gene sequence of the obtained amplified fragment to check if the gene sequence matches the *Glu-B3h* gene sequence (GenBank Accession No. 369717: SEQ ID NO: 18). Alternatively, the confirmation may be made by SDS-PAGE as in the case of the *Glu-B3b* allele. Known wheat varieties possessing the *Glu-B3h* allele include "Horoshiri-Komugi", "Haruyutaka", and so forth (Non Patent Literature 13).

Whether or not wheat possesses the *Glu-B3i* allele can be determined by examining whether the wheat possesses a sequence specific to *Glu-B3i* in the gene sequence described in Non Patent Literature 14 (GenBank Accession No. EU369720: SEQ ID NO: 19). This identification may be made by: performing PCR using SB9F and SB9R primers described in Non Patent Literature 14 (SB9F: SEQ ID NO: 33 and SB9R: SEQ ID NO: 34); and examining whether or not an amplified fragment with a desired length is obtained. Alternatively, the identification may be made by: performing PCR using LB3F and LB3R primers or LB4F and LB4R primers described in Non Patent Literature 14 (LB3F: SEQ ID NO: 35, LB3R: SEQ ID NO: 36, LB4F: SEQ ID NO: 37, and LB4R: SEQ ID NO: 38), and analyzing the gene sequence of the resulting amplified fragment to examine whether or not the gene sequence matches the *Glu-B3i* gene sequence (GenBank Accession No. EU369718: SEQ ID NO: 18, EU369720: SEQ ID NO: 17, or EU369714: SEQ ID NO: 19). Alternatively, the confirmation may be made by SDS-PAGE as in the case of the *Glu-B3b* allele. Known wheat varieties possessing the *Glu-B3i* allele include "Norin 61", "Minami no Kaori", "Iwai no Daichi", and so forth (Non Patent Literature 15).

The gene sequences and primer sequences usable for the above allele identifications are shown in the sequence listings in Tables 2 and 3.

**[Table 2]**

| Allele | GenBank Accession No. | Sequence Listing |
|---|---|---|
| *Glu-D1d* | X12928 | SEQ ID NO: 13 |
| *Glu-B3b* | EU369719 | SEQ ID NO: 14 |
| | EU369700 | SEQ ID NO: 15 |
| *Glu-B3h* | EU369717 | SEQ ID NO: 16 |
| *Glu-B3i* | EU369720 | SEQ ID NO: 17 |
| | EU369718 | SEQ ID NO: 18 |
| | EU369714 | SEQ ID NO: 19 |

**[Table 3]**

| Allele | Primer | Sequence Listing |
|---|---|---|
| *Glu-D1d* | Dx_F | SEQ ID NO: 20 |
| | Dx5_F | SEQ ID NO: 21 |
| | Dx_R | SEQ ID NO: 22 |
| *Glu-B3b* | SB2F | SEQ ID NO: 23 |
| | SB2R | SEQ ID NO: 24 |
| | LB1F | SEQ ID NO: 25 |
| | LB1R | SEQ ID NO: 26 |
| | LB4F | SEQ ID NO: 27 |
| | LB4R | SEQ ID NO: 28 |
| *Glu-B3h* | SB8F | SEQ ID NO: 29 |
| | SB8R | SEQ ID NO: 30 |
| | LB3F | SEQ ID NO: 31 |
| | LB3R | SEQ ID NO: 32 |
| *Glu-B3i* | SB9F | SEQ ID NO: 33 |
| | SB9R | SEQ ID NO: 34 |
| | LB3F | SEQ ID NO: 35 |
| | LB3R | SEQ ID NO: 36 |
| | LB4F | SEQ ID NO: 37 |
| | LB4R | SEQ ID NO: 38 |

The wheat flour of the present invention is wheat flour (GA-SX/GD1d wheat flour) obtained by milling harvested GA-SX/GD1d wheat mentioned above. The wheat flour is preferably wheat flour (GA-SX/GD1d/GB3b wheat flour) obtained by milling harvested GA-SX/GD1d/GB3b wheat or preferably wheat flour (GA-SX/GD1d/GB3h wheat flour) obtained by milling harvested GA-SX/GD1d/GB3h wheat. The milling method is not particularly limited, and any of general milling methods used to produce wheat flour from a harvested product (grains or seeds) of a conventional wheat variety may be used. The form of the wheat flour is not particularly limited, and for example, it may be wheat flour from which components such as "bran" are removed through a normal milling process, or may be whole wheat flour without any separation.

The wheat flour of the present invention may also be provided as a cereal flour composition mixed with another type of wheat flour or non-wheat flour. Examples of the other types of wheat flour include wheat flour such as strong flour, medium flour, and weak flour or wheat-derived flour not classified as the above types. Examples of non-wheat flour include, but are not limited to, flours derived from grains such as rice, rye, barley, corn, buckwheat, soybeans, millet, foxtail millet, and amaranth.

In the cereal flour composition of the present invention, the content of the wheat flour of the present invention is preferably 5 to 100% by mass, more preferably 25 to 100% by mass, and even more preferably 50 to 100% by mass, based on the total amount of the cereal flour composition. Most preferably, it is 100% by mass.

Having the high molecular weight glutenin gene *Glu-D1* of the d-type allele (GA-SX/GD1d), the wheat flour and the cereal flour composition of the present invention make it possible to maintain deterioration reduction at a level comparable to the conventional GA-SX wheat flour and also provide products with improved texture by making improvement in the stickiness and the meltability in the mouth, which are considered as the problem in foods, particularly bakery foods, made from conventional GA-SX wheat flour or a cereal flour composition containing the GA-SX wheat flour. Further having the low molecular weight glutenin gene *Glu-B3* of the b-type or h-type allele, the wheat flour and the cereal flour composition make it possible to provide a product with even more improved texture.

The wheat flour and the cereal flour composition in the present invention are usable in productions of various types of foods made by using wheat flour and a cereal flour composition containing the wheat flour. Examples of such foods include bakery foods such as bread, cakes, baked goods, and pizzas; noodles such as udon and Chinese noodles; fried foods such as tempura and fried foods; baked foods such as dumplings, spring rolls, and shumai (their wrappers containing wheat flour); and fish pastes such as kamaboko and chikuwa. Preferably, the foods are bakery foods. The foods may be produced using a conventional production method, except for the use of the wheat flour or the cereal flour composition of the present invention.

Examples of the bakery products include: breads such as white bread, baguettes, rolls, and sweet buns; fried breads such as yeast donuts; steamed buns; pizzas such as pizza pies; cakes such as sponge cakes; and baked sweets such as cookies and biscuits. The bakery food of the present invention may be produced, for example, by kneading a mixture of the wheat flour or the cereal flour composition of the present invention with various auxiliary ingredients commonly used in the production of the bakery food, such as a chemical leavening agent such as baking soda, yeast, yeast food, salt, sugar, oil and/or fat, egg, dairy product, and water, to make a dough, which is then proofed or is directly baked or deep-fried as it is. Additives such as vitamins and minerals may be added as needed. Conventional production methods may be used to produce the bakery foods of the present invention.

### [Examples]

Hereinafter, the present invention will be described in further details based on Examples, but the present invention should not be limited to these Examples.

### <1. Development of Wheat>

To develop the wheat of the present invention, wheat having the alleles listed in the following table was used.

| Wheat Name | High Molecular Weight Glutenin | Low Molecular Weight Glutenin | Starch Synthase | | | | | |
|---|---|---|---|---|---|---|---|---|
| | *Glu-D1* | *Glu-B3* | *GBSSI-A1* | *GBSSI-B1* | *GBSSI-D1* | *SSIIa-A1* | *SSIIa-B1* | *SSIIa-D1* |
| Wheat (i) | d | h | a | b | a | a | a | A |
| Wheat (ii) | Not investigated | Not investigated | a | b | b | a | b | B |
| wheat (iii) | d | b | a | b | a | a | a | A |
| Wheat (iv) | Not investigated | Not investigated | b | b | b | a | a | A |
| wheat (v) | Not investigated | Not investigated | a | a | a | b | b | B |
| wheat (vi) | d | b | a | b | a | a | a | A |
| Wheat (vii) | d | i | b | b | b | a | a | A |
| Wheat (viii) | f | i | a | a | a | b | b | B |

### (1) Creation of SK wheat

Through continuous backcrossing, the *GBSSI-D1b, SSIIa-B1b*, and *D1b* alleles possessed by wheat (ii) were introduced into wheat (i), thereby creating SK wheat. The backcrossing is a breeding method in which a progeny obtained by crossing two varieties is crossed again with one of the parent varieties (recurrent parent). Then, the continuous backcrossing refers to recurrent crossing with the same recurrent parent. This continuous backcrossing is a method often used, for example, in a case where a gene (for example, disease resistance or the like) not possessed by a desired elite variety is efficiently introduced from another variety into this elite variety, while many of the traits of the elite variety are maintained. The parent used in the recurrent crossing is called a recurrent parent, and the parent that donates the desired gene is called a donor parent. In the creation of the SK wheat, the continuous backcrossing was performed using the wheat (i) as the recurrent parent and the wheat (ii) as the donor parent. In each cycle of the continuous backcrossing, the obtained individuals were examined to determine the alleles at the *GBSSI-D1*, *SSIIa-B1*, and *D1* gene loci. Individuals heterozygous for the a and b alleles at all the three gene loci were selected and used in the next backcrossing. After the backcrossing, the selected individuals were self-propagated to obtain the progeny, and individuals possessing homozygous alleles at each of the eight gene loci as specified in Table 1 were selected.

### (2) Creation of HK wheat

The continuous backcrossing using wheat (iii) as the recurrent parent and wheat (iv) as the donor parent was performed, and wheat (iii)-A, in which *GBSSI-A1b* and *GBSSI-D1b* alleles were introduced into the wheat (iii), was selected. In the same manner, the continuous backcrossing of wheat (iii) with wheat (v) used as the donor parent was performed, and wheat (iii)-B, in which *SSIIa-A1b*, *B1b*, and *D1b* alleles were introduced into the wheat (iii) was selected. These wheat species (iii)-A and (iii)-B were crossed, followed by genetic fixation using the doubled haploid breeding method (Non Patent Literature 16), and HK wheat having homozygous alleles at each of the eight gene loci as specified in Table 1 was selected from obtained DH population (DH population).

### (3) Creation of N1-1 Wheat

Wheat (vii) and wheat (viii) were crossed, and the backcrossing of the obtained F₁ generation individuals with the wheat (vi) as the recurrent parent was performed. From this progeny, several individuals were selected which had homozygous *GBSSI-A1a*, *B1b*, and *SSIIa-A1a* alleles, and were heterozygous for *GBSSI-D1*, *SSIIa-B1*, and *D1*. A next-generation population was obtained from these individuals, followed by genetic fixation using the doubled haploid breeding method to obtain the DH population. From this DH population, N1-1 wheat was obtained which had homozygous alleles at each of the eight gene loci as specified in Table 1.

In the above crossbreeding and selection process, the allele at each gene locus was determined according to the following method.

The allele determination at the *GBSSI-A1* and *D1* gene loci was performed according to the method described in Non Patent Literature 17. The determination at the GBSSI-B1 gene locus was performed according to the method described in Non Patent Literature 8.

The allele determination at the *SSIIa-A1*, *B1*, and *D1* gene loci was performed according to the method described in Non Patent Literature 9.

The determination of the *Glu-D1d* allele was performed according to the method described in Non Patent Literature 11.

The determination of the *Glu-B3b* and *B3h* alleles was performed according to the method described in Non Patent Literature 14.

The determination of the *Glu-B3i* allele was performed by sequencing the sequence of a fragment amplified using the LB3F and LB3R primers described in Non Patent Literature 14 under the PCR conditions specified in the same Literature, and examining its homology to the *Glu-B3i* allele sequence (GenBank Accession No. EU 369718, SEQ ID NO: 18).

Here, DFBI wheat (MK 5-5 NIL wheat described in Non Patent Literature 2), which is conventional GA-SX wheat, was used as a comparative example. The genotype of each wheat species is specified in Table 1 below.

**Table 1 Genotype (allele) of Wheat Variety**

| Wheat Name | High Molecular Weight Glutenin | Low Molecular Weight Glutenin | Starch Synthase | | | | | |
|---|---|---|---|---|---|---|---|---|
| | *Glu-D1* | *Glu-B3* | *GBSSI-A1* | *GBSSI-B1* | *GBSSI-D1* | *SSIIa-A1* | *SSIIa-B1* | *SSIIa-D1* |
| SK | D | h | a | b | b | a | b | b |
| HK | D | b | a | b | b | a | b | b |
| N 1-1 | D | i | a | b | b | a | b | b |
| DFBI | F | i | a | b | b | a | b | b |

### <2. Preparation of Wheat Flour>

The harvested wheat grain was ground using an experimental mill manufactured by Bühler. After the wheat grain was adjusted to a moisture content of 14% with addition of water and then left to stand overnight, the wheat grain was put into the experimental mill and ground to prepare 60% extraction flour.

### <3. Bread Making Test>

Using the above wheat flour, two loaves of white bread were produced according to a 70% standard sponge and dough method. A mixture obtained by using 70 parts by mass out of 100 parts by mass of the wheat flour and adding 2.3 parts by mass of yeast, 0.1 parts by mass of yeast food, and 40 parts by mass of water was mixed using a commercially available bread mixer (product name: SK 200 manufactured by SK Mixer Co., Ltd.) at low speed for 2 minutes and then at medium speed for 2 minutes to obtain a dough. This dough was proofed for 4 hours in an environment at a temperature of 27°C and a relative humidity of 75%. To this dough, 30 parts by mass of the wheat flour, 5 parts by mass of sugar, 2 parts by mass of salt, 5 parts by mass of shortening, 2 parts by mass of skim milk powder, and an appropriate amount of water were added. The water was added in such a preset amount as to equalize dough properties (elasticity and stickiness) among test groups. The dough was mixed at low speed for 2 minutes, at medium speed for 3 minutes, and at high speed for 1 minute, then scraped down, and mixed again at low speed for 1 minute, at medium speed for 3 minutes, and at high speed for 7 minutes. The resultant dough was proofed for 20 minutes and then divided into 460 g portions, which were shaped and placed in baking pans. After that, the dough portions were proofed under the conditions at 38°C and 85% humidity, and baked when the proofed dough portions reached 1 cm above the tops of the pans (reel oven, 210°C, 25 minutes). After baking, the loaves of white bread were taken out from the pans, allowed to cool at room temperature for 1 hour, and then sealed in polyethylene bags. One of the loaves was left to stand at room temperature until the next day, and the other loaf was left to stand until the third day.

Each of the loaves was sliced with a 12.5 mm thickness before tasting test and evaluated by 10 panelists according to the criteria shown in the evaluation criteria table (Table 2). The next-day evaluation was conducted by using the next-day DFBI evaluation as a baseline (3.0 points). The third-day evaluation was conducted by using, as a baseline (3.0 points), the evaluation of DFBI slices frozen on the next day after baking and then thawed at room temperature for 4 hours on the evaluation day.

**Table 2 Evaluation Criteria Table**

| | | |
|---|---|---|
| Softness | 5 points | Soft |
| | 4 points | Fairly Soft |
| | 3 points | Comparable to Control |
| | 2 points | Slightly Hard |
| | 1 point | Hard |
| Stickiness | 5 points | Weakly Sticky |
| | 4 points | Slightly-Weakly Sticky |
| | 3 points | Comparable to Control |
| | 2 points | Slightly-Strongly Sticky |
| | 1 point | Strongly Sticky |
| Meltability in Mouth | 5 points | Meltable in Mouth |
| | 4 points | Slightly Meltable in Mouth |
| | 3 points | Comparable to Control |
| | 2 points | Slightly-Poorly Meltable in Mouth |
| | 1 point | Poorly Meltable in Mouth |

### <Production Examples>

White bread was produced using the wheat flour prepared from SK, HK, N1-1, and DFBI, and sensory evaluation was conducted on the next day and the third day after baking. The results are shown in Table 3.

**Table 3 Sensory Evaluation Results**

| | Example 1 | Example 2 | Example 3 | Comparative Example 1 |
|---|---|---|---|---|
| Flour | SK | HK | N1-1 | DFBI |
| *Glu-B3* | h | b | I | I |
| *Glu-D1* | d | d | D | F |
| Softness (Next Day) | 3.2 | 3.2 | 3.1 | 3.0 |
| Stickiness (Next Day) | 3.9 | 3.8 | 3.6 | 3.0 |
| Meltability in Mouth (Next Day) | 4.0 | 3.8 | 3.6 | 3.0 |
| Softness (3rd Day) | 3.0 | 3.0 | 2.9 | 2.6 |
| Stickiness (3rd Day) | 4.1 | 4.0 | 3.8 | 3.1 |
| Meltability in Mouth (3rd Day) | 4.0 | 3.9 | 3.8 | 3.3 |

Regarding the softness, SK, HK, and N1-1 were comparable to DFBI. On the other hand, regarding the stickiness and the meltability in the mouth, all the three samples SK, HK, and N1-1 scored higher than DFBI on both the next day and the third day after baking. Among the three samples, HK scored higher than N1-1 and SK scored even higher than HK on both the next day and the third day after baking. The above results show that, having *Glu-D1d*, the GA-SX wheat achieved significant improvements in the stickiness and the meltability in the mouth of the white bread. In addition, this effect was greatest when the GA-SX wheat additionally had *Glu-B3h*, and became smaller with *Glu-B3b* and *B3i* in this order.

## Claims

1. Wheat flour obtained by milling harvested wheat which does not lack GBSSI-A1 enzyme activity, lacks GBSSI-B1 and GBSSI-D1 enzyme activity, and lacks enzyme activity of any two of SSIIa-A1, SSIIa-B1, and SSIIa-D1, and in which a high molecular weight glutenin gene *Glu-D1* is a d-type allele (GA-SX/GD1d wheat flour).

2. The wheat flour according to claim 1, wherein a low molecular weight glutenin gene *Glu-B3* is a b-type allele (GA-SX/GD1d/GB3b wheat flour).

3. The wheat flour according to claim 1, wherein a low molecular weight glutenin gene *Glu-B3* is an h-type allele (GA-SX/GD1d/GB3h wheat flour).

4. A cereal flour composition comprising the wheat flour according to any one of claims 1 to 3.

5. A method for producing a food product using the wheat flour according to any one of claims 1 to 3.

6. The method according to claim 5, wherein the food product is a bakery food product.
